# EUROPEAN PATENT APPLICATION

(11) **EP 4 552 683 A1**
(43) Date of publication of application: **14.05.2025**
(21) Application number: 23834564.9
(22) Date of filing: 05.06.2023
(51) Int. Cl.: A61M 60/237, A61M 60/216, A61M 60/829

(54) **DRIVING DEVICE AND BLOOD PUMP**

(30) Priority: 08.07.2022 CN 202210800097
(71) Applicant: Shenzhen Core Medical Technology Co., Ltd., Shenzhen, Guangdong 518000 (CN)
(72) Inventor: ZHU, Yicheng, Shenzhen, Guangdong 518000 (CN); YU, Shunzhou, Shenzhen, Guangdong 518000 (CN)
(74) Representative: Murgitroyd & Company
(86) International application number: PCT/CN2023/098337
(87) International publication number: WO 2024/007790

(57) **Abstract**

The driving device (20) comprises a housing assembly (100), a rotating shaft (200), a rotor (400), and a stator (300). An accommodating cavity (150) of the housing assembly (100) has a first cavity wall (151) and a second cavity wall (152). A protruding portion (220) of the rotating shaft (200) has a first surface (221) and a second surface (222). The first surface (221) faces the first cavity wall (151). The second surface (222) faces the second cavity wall (152). The area of the first surface (221) is greater than the area of the second surface (222). The area of the first surface (221) is less than or equal to the area of the first cavity wall (151). There is an attraction force between the stator (300) and the rotor (400), and the attraction force enables the first surface (221) to abut against the first cavity wall (151).

## Description

This application claims priority to Chinese patent application No. 202210800097.9, July 8, 2022, the content of which is incorporated herein by reference in its entirety.

### TECHNICAL FIELD

The present application relates to the technical field of medical devices, and in particular, to a driving device and a blood pump including the driving device.

### BACKGROUND

An intravascular blood pump is a blood pumping device that can be inserted into the patient's heart through the patient's blood vessels. The intravascular blood pump is placed in an opening of the heart valve, so that blood can flow through the blood pump and enter the artery blood vessel. The blood pump includes a driving device and an impeller. The impeller is fixed to a rotating shaft of the driving device and is driven to rotate by the rotation of the rotating shaft. However, the rotating shaft of a conventional blood pump is severely worn during the use of the blood pump and is difficult to start up, which affects the use of the blood pump.

### SUMMARY

Accordingly, the present application provides a driving device and a blood pump, which can reduce the wear of the rotating shaft and are easy to start up.

An embodiment of a first aspect of the present application provides a driving device configured to drive an impeller to rotate. The driving device includes:
a housing assembly provided with a receiving cavity, wherein the receiving cavity has a first cavity wall and a second cavity wall that face each other and are spaced apart from each other;
a rotating shaft configured to be connected to the impeller, the rotating shaft including a straight shaft part and a raised part that are connected to each other, wherein the raised part protrudes from the straight shaft part in a circumferential direction of the straight shaft part; the raised part is rotatably received in the receiving cavity; the raised part is located between the first cavity wall and the second cavity wall and has a first surface facing the first cavity wall and a second surface facing the second cavity wall; an area of the first surface is greater than an area of the second surface, and the area of the first surface is less than or equal to an area of the first cavity wall;
a rotor fixedly connected to the straight shaft part; and
a stator configured to drive the rotor to rotate. An attractive force is generated between the stator and the rotor, and the attractive force enables the first surface to abut against the first cavity wall.

An embodiment of a second aspect of the present application provides a blood pump, including an impeller and a driving device. The driving device includes:
a housing assembly provided with a receiving cavity, wherein the receiving cavity has a first cavity wall and a second cavity wall that face each other and are spaced apart from each other;
a rotating shaft including a straight shaft part and a raised part that are connected to each other, wherein the straight shaft part is connected to the impeller; the raised part protrudes from the straight shaft part in a circumferential direction of the straight shaft part; the raised part is rotatably received in the receiving cavity; the raised part is located between the first cavity wall and the second cavity wall and has a first surface facing the first cavity wall and a second surface facing the second cavity wall; an area of the first surface is greater than an area of the second surface, and the area of the first surface is less than or equal to an area of the first cavity wall;
a rotor fixedly connected to the straight shaft part; and
a stator configured to drive the rotor to rotate. An attractive force is generated between the stator and the rotor, and the attractive force enables the first surface to abut against the first cavity wall.

The details of one or more embodiments of the present application are set forth in the following drawings and detailed description. Other features, objects, and advantages of the present application will become apparent from the detailed description, drawings, and claims.

### BRIEF DESCRIPTION OF THE DRAWINGS

In order to illustrate the technical solutions in the embodiments of the present application more clearly, the drawings required to be used in the description of the embodiments or the prior art will be briefly introduced below. Apparently, the drawings described below are only for some embodiments of the present application. For ordinary skills in the art, other drawings can be obtained based on these drawings without creative efforts.
FIG. 1 is a perspective schematic view of a blood pump according to a first embodiment.
FIG. 2 is a partial sectional view of the blood pump shown in FIG. 1.
FIG. 3 is another partial sectional view of the blood pump shown in FIG. 1.
FIG. 4 is an exploded schematic view of the blood pump shown in FIG. 1.
FIG. 5 is an exploded schematic view of a rotating shaft, a first shaft sleeve and a second shaft sleeve of the blood pump shown in FIG. 2.
FIG. 6 is a perspective schematic view of a stator of the blood pump shown in FIG. 2.
FIG. 7 is a front view of a rotor of the blood pump shown in FIG. 2.
FIG. 8 is a schematic sectional view of the rotor shown in FIG. 7.
FIG. 9 is an exploded schematic view of the rotor shown in FIG. 7;
FIG. 10 is a sectional view of a blood pump according to a second embodiment.

### DETAILED DESCRIPTION

In order to make the purpose, technical solution and advantages of the present application more clearly understood, the present application is further described in detail below in conjunction with the accompanying drawings, i.e., embodiments. It should be understood that the specific embodiments described herein are only used to explain the present application and are not used to limit the present application.

It should be noted that when an element is referred to as being "fixed to" or "disposed on" another element, it can be directly on the other element or indirectly on the other element. When an element is referred to as being "connected to" another element, it can be directly connected to the other element or indirectly connected to the other element.

In addition, the terms "first" and "second" are used for descriptive purposes only and should not be understood as indicating or implying relative importance or implicitly indicating the number of the indicated technical features. Thus, a feature defined by "first" or "second" may explicitly or implicitly include one or more of the features. In the description of this application, the meaning of "plurality" is two or more, unless otherwise clearly and specifically defined.

In order to illustrate the technical solution of the present application, a description is given below in conjunction with specific drawings and embodiments.

In this document, a "proximal end" is defined as an end closer to an operator or doctor, and a "distal end" is defined as an end farther from the operator or doctor.

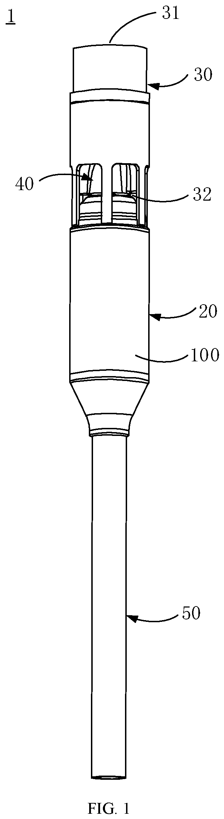

Referring to FIG. 1, a blood pump 1 according to a first embodiment of the present application includes a driving device 20, a cannula 30, an impeller 40, and a catheter 50. The cannula 30 is connected to the distal end of the driving device 20. The catheter 50 is connected to the proximal end of the driving device 20. The impeller 40 is rotatably disposed in the cannula 30. The impeller 40 is connected to the driving device 20. The driving device 20 can drive the impeller 40 to rotate to enable the blood pumping function of the blood pump 1.

Specifically, the cannula 30 has a liquid inlet 31 and a liquid outlet 32. The liquid outlet 32 is closer to the driving device 20 than the liquid inlet 31. That is, the liquid outlet 32 is located at the proximal end of the cannula 30, and the liquid inlet 31 is located at the distal end of the cannula 30. The liquid outlet 32 is located on the wall of the cannula 30. The impeller 40 is adjacent to the liquid outlet 32. In an embodiment, the cannula 30 extends through a heart valve, such as an aortic valve, so that the liquid inlet 31 is located in the heart, and the liquid outlet 32 and the driving device 20 are located in a blood vessel such as the aorta outside the heart. When the impeller 40 rotates, the blood flows into the cannula 30 via the liquid inlet 31, and then flows out of the cannula 30 via the liquid outlet 32 to enter the blood vessel such as the aorta.

The catheter 50 is connected to an end of the driving device 20 away from the cannula 30. The catheter 50 is configured to accommodate various supply lines, such as a cleaning line for passing a flushing liquid into the driving device 20, a conducting wire for supplying power to the driving device 20, or a supporting component for supporting the catheter 50.

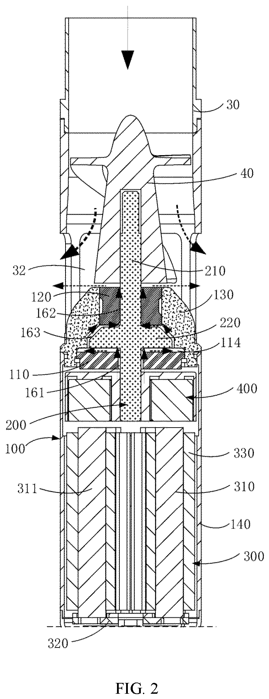

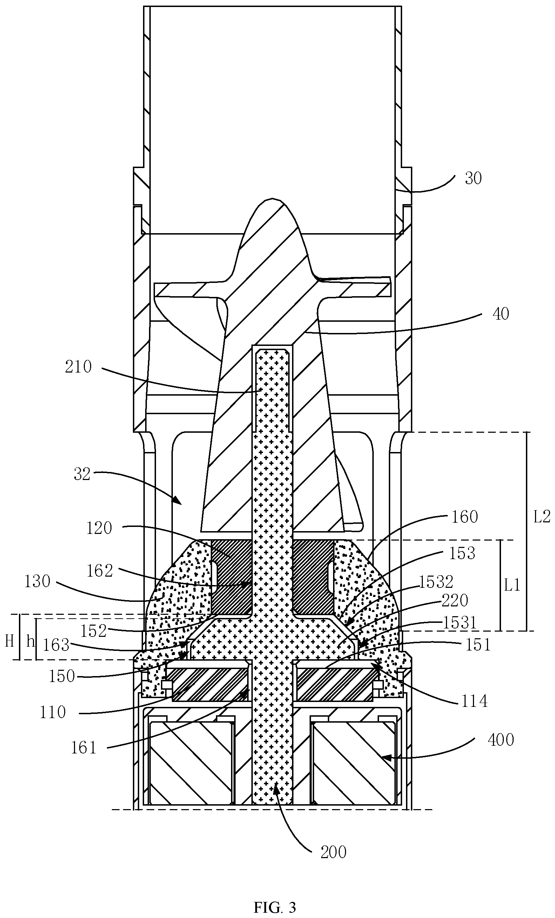

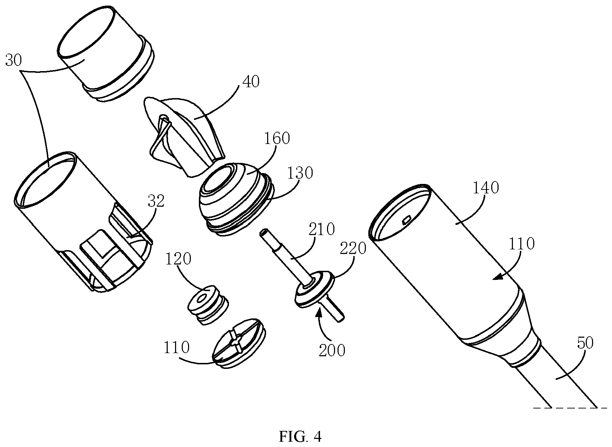

Still referring to FIGS. 2 to 4, the driving device 20 includes a housing assembly 100, a rotating shaft 200, a stator 300, and a rotor 400. The rotating shaft 200 can be rotatably mounted on the housing assembly 100. The rotating shaft 200 has a portion received in the housing assembly 100, and another portion extending into the cannula 30 and fixedly connected to the impeller 40. The stator 300 and the rotor 400 are both received in the housing assembly 100. The rotor 400 is fixedly connected to the rotating shaft 200. The stator 300 can drive the rotor 400 to rotate. The rotor 400 can drive the rotating shaft 200 to rotate. The impeller 40 can rotate with the rotating shaft 200 to enable the blood pumping function of the blood pump 1.

The proximal end and the distal end of the housing assembly 100 are fixedly connected to the catheter 50 and the cannula 30, respectively. The conducting wire in the catheter 50 extends into the housing assembly 100 and is electrically connected to the stator 300 to supply power to the stator 300. Specifically, the housing assembly 100 includes a first shaft sleeve 110, a second shaft sleeve 120, a shaft tube 130, and a pump housing 140. The first shaft sleeve 110 and the second shaft sleeve 120 are fixedly received in the shaft tube 130. An end of the shaft tube 130 is fixedly connected to the pump housing 140, and another end of the shaft tube 130 is fixedly connected to the cannula 30. An end of the pump housing 140 away from the shaft tube 130 is fixedly connected to the catheter 50. An end of the rotating shaft 200 away from the impeller 40 is received in the pump housing 140. The rotor 400 and the stator 300 are received in the pump housing 140. In an embodiment, the first shaft sleeve 110, the second shaft sleeve 120, the shaft tube 130, and the pump housing 140 are separate before assembly, that is, the housing assembly 100 is formed by assembling the first shaft sleeve 110, the second shaft sleeve 120, the shaft tube 130 and the pump housing 140 that are separate. It can be understood that in other embodiments, the first shaft sleeve 110, the second shaft sleeve 120, the shaft tube 130 and the pump housing 140 can also be an integrally formed structure.

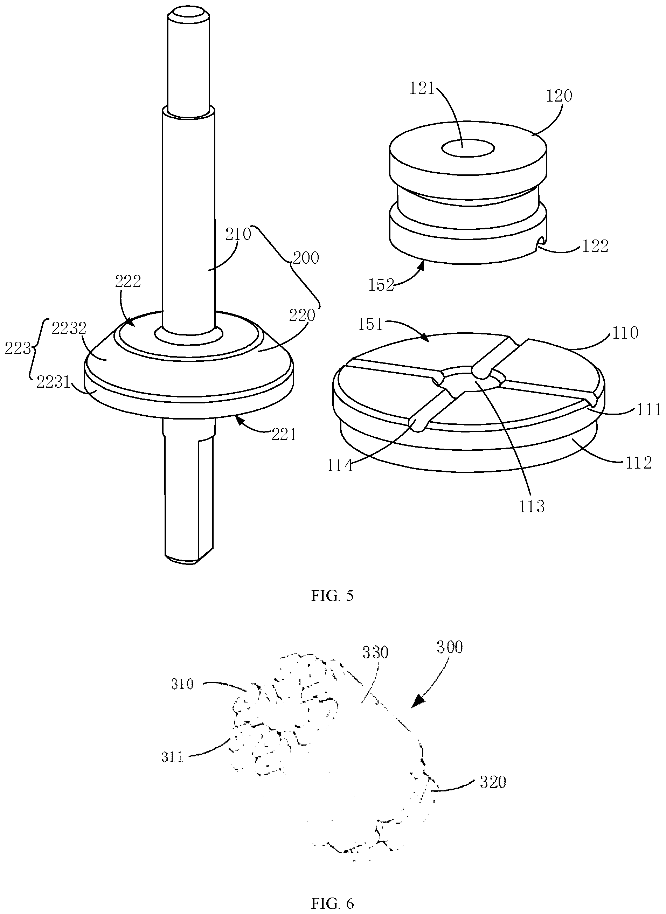

Referring to FIG. 5, in some embodiments, the first shaft sleeve 110 can be fixedly connected to the shaft tube 130 by adhering. In some embodiments, the first shaft sleeve 110 includes a large plate 111 and a small plate 112. The large plate 111 and the small plate 112 are coaxially arranged. The outer diameter of the large plate 111 is greater than the outer diameter of the small plate 112. A gap between the small plate 112 and the shaft tube 130 can form an adhesive-injection space. After an adhesive solidifies in the adhesive-injection space, the entire first shaft sleeve 110 is adhered to the shaft tube 130. The second shaft sleeve 120 can also be fixedly connected to the shaft tube 130 by adhering.

The first shaft sleeve 110 and the second shaft sleeve 120 are spaced apart in the axial direction of the shaft tube 130. The first shaft sleeve 110 is farther away from the impeller 40 than the second shaft sleeve 120. The shaft tube 130, the first shaft sleeve 110 and the second shaft sleeve 120 jointly enclose a receiving cavity 150. The receiving cavity 150 is located between the first shaft sleeve 110 and the second shaft sleeve 120.

The receiving cavity 150 has a first cavity wall 151, a second cavity wall 152, and a side cavity wall 153. The side cavity wall 153 connects the first cavity wall 151 and the second cavity wall 152, so that the first cavity wall 151, the second cavity wall 152 and the side cavity wall 153 jointly define the boundary of the receiving cavity 150. The first cavity wall 151 is located on the first shaft sleeve 110, the second cavity wall 152 is located on the second shaft sleeve 120, and the side cavity wall 153 is located on the shaft tube 130. The first cavity wall 151 and the second cavity wall 152 face each other and are spaced apart. The first cavity wall 151 faces the impeller 40, and the second cavity wall 152 faces away from the impeller 40. Specifically, the first cavity wall 151 and the second cavity wall 152 are perpendicular to the axial direction of the shaft tube 130. That is, the first cavity wall 151 and the second cavity wall 152 are parallel to each other. At least a portion of the surface of the first shaft sleeve 110 facing the impeller 40 forms the first cavity wall 151. At least a portion of the surface of the second shaft sleeve 120 facing away from the impeller 40 forms the second cavity wall 152. In the illustrated embodiment, the area of the first cavity wall 151 is greater than that of the second cavity wall 152.

The first cavity wall 151 is provided with a first through hole 113. The first through hole 113 is in communication with the receiving cavity 150. The first through hole 113 extends in the axial direction of the first shaft sleeve 110 and extends through the entire first shaft sleeve 110. In the illustrated embodiment, the first cavity wall 151 is substantially circular, and the first through hole 113 is located at the center of the first cavity wall 151.

The first cavity wall 151 is further provided with a first guide groove 114. The first guide groove 114 is in communication with the first through hole 113 and the receiving cavity 150. In the illustrated embodiment, the first guide groove 114 extends in the radial direction of the first shaft sleeve 110. At least three first guide grooves 114 are provided. The at least three first guide grooves 114 are evenly spaced apart in the circumferential direction of the first through hole 113. In some embodiments, an end of each first guide groove 114 extends to the first through hole 113 and is in communication with the first through hole 113, and another end of each first guide groove 114 extends to the edge of the first cavity wall 151. In other embodiments, the end of the first guide groove 114 away from the first through hole 113 does not extend to the edge of the first cavity wall 151, and in this case, the end of the first guide groove 114 away from the first through hole 113 is at a certain distance from the edge of the first cavity wall 151.

The second cavity wall 152 is provided with a second through hole 121. The second through hole 121 is in communication with the receiving cavity 150. The second through hole 121 extends in the axial direction of the second shaft sleeve 120 and extends through the entire second shaft sleeve 120. In the illustrated embodiment, the second cavity wall 152 is substantially circular, and the second through hole 121 is located at the center of the second cavity wall 152.

The second cavity wall 152 is further provided with a second guide groove 122. The second guide groove 122 is in communication with the second through hole 121 and the receiving cavity 150. In the illustrated embodiment, the second guide groove 122 extends in the radial direction of the second shaft sleeve 120. The second guide groove 122 may be provided as the first guide groove 114, which will not be repeatedly described herein. In some embodiments, an end of the second guide groove 122 extends to the second through hole 121 and is in communication with the second through hole 121, and another end of the second guide groove 122 extends to the edge of the second cavity wall 152. In other embodiments, the end of the second guide groove 122 away from the second through hole 121 does not extend to the edge of the second cavity wall 152. In this case, the end of the second guide groove 122 away from the second through hole 121 is at a certain distance from the edge of the second cavity wall 152.

The pump housing 140 is substantially cylindrical and is in communication with the receiving cavity 150 through the first through hole 113. The flushing liquid entering the pump housing 140 may enter the receiving cavity 150 via the first through hole 113 and flow out of the housing assembly 100 via the second through hole 121.

Specifically, the rotating shaft 200 rotatably extends through the first through hole 113, the second through hole 121 and the receiving cavity 150. The rotating shaft 200 includes a straight shaft part 210 and a raised part 220 that are connected to each other.

A portion of the straight shaft part 210 is received in the housing assembly 100, and another portion of the straight shaft part 210 extends into the cannula 30 and is fixedly connected to the impeller 40. The straight shaft part 210 rotatably extends through the first through hole 113, the second through hole 121, and the receiving cavity 150. Specifically, the cross section of the portion of the straight shaft part 210 received in the first through hole 113 and the second through hole 121 is circular, and the first through hole 113 and the second through hole 121 are substantially circular holes.

In the illustrated embodiment, a first gap 161 is formed between the straight shaft part 210 and the hole wall of the first through hole 113. The first gap 161 can be understood as a portion of the first through hole 113 that is not filled with the straight shaft part 210. The cleaning liquid in the pump housing 140 can enter the receiving cavity 150 via the first gap 161. A second gap 162 is formed between the straight shaft part 210 and the hole wall of the second through hole 121. The second gap 162 can be understood as a portion of the second through hole 121 that is not filled with the straight shaft part 210. The flushing liquid in the receiving cavity 150 can flow out of the housing assembly 100 via the second gap 162. Specifically, the width of at least a portion of the second gap 162 is less than the width of the first gap 161.

The end of the hole wall of the first through hole 113 adjacent to the receiving cavity 150 is provided with a chamfer. Such design can reduce the contact area between the straight shaft part 210 and the hole wall of the first through hole 113 when the rotating shaft 200 shakes and is in contact with the hole wall of the first through hole 113, thereby reducing the friction received by the straight shaft part 210. The chamfer can also guide the assembling, reduce the interference and assembling resistance encountered by the rotating shaft 200 during the assembling process, and improve the assembling efficiency of the rotating shaft 200. The end of the hole wall of the second through hole 121 adjacent to the receiving cavity 150 is provided with a chamfer. Such design can reduce the contact area between the straight shaft part 210 and the hole wall of the second through hole 121 when the rotating shaft 200 shakes and is in contact with the hole wall of the second through hole 121, thereby reducing the friction received by the straight shaft part 210. This chamfer can also guide the assembling, reduce the interference and assembling resistance encountered by the rotating shaft 200 during the assembling process, and improve the assembling efficiency of the rotating shaft 200.

The raised part 220 protrudes from the straight shaft part 210 in the circumferential direction thereof. The raised part 220 can be rotatably received in the receiving cavity 150. The raised part 220 is located between the first cavity wall 151 and the second cavity wall 152. The first cavity wall 151 and the second cavity wall 152 can each abut against the raised part 220, to limit the maximum vibration amplitude of the rotating shaft 200 in the axial direction. Specifically, a size of a cross-section of the raised part 220 is greater than a diameter of the first through hole 113, and also greater than a diameter of the second through hole 121, so that the raised part 220 is limited in the receiving cavity 150, and the raised part 220 cannot enter the first through hole 113 and the second through hole 121. In the illustrated embodiment, the raised part 220 is annular and is fixedly sleeved on the straight shaft part 20. The outer diameter of the raised part 220 is greater than the diameter of the straight shaft part 210. The axis of the raised part 220 coincides with the axis of the straight shaft part 210. The outer diameter of the raised part 220 is greater than the hole size of the first through hole 113 and also greater than the hole size of the second through hole 121.

The raised part 220 has a first surface 221 and a second surface 222. The first surface 221 and the second surface 222 are spaced apart along the axis of the straight shaft part 210. The first surface 221 faces the first cavity wall 151. The second surface 222 faces the second cavity wall 152. The first cavity wall 151 can abut against the first surface 221, and the second cavity wall 152 can abut against the second surface 222 so that the maximum vibration amplitude of the rotating shaft 200 in the axial direction. In the illustrated embodiment, the first surface 221 and the second surface 222 are both perpendicular to the axis of the straight shaft part 210, and the first cavity wall 151 and the second cavity wall 152 are parallel to the first surface 221 and the second surface 222 respectively. The outer contours of the first surface 221 and the second surface 222 are both circular. The first surface 221 and the second surface 222 are coaxial with the axis of the straight shaft part 210, that is, the axis of the straight shaft part 210 extends through the center of the circle where the first surface 221 is located and the center of the circle where the second surface 222 is located.

The area of the first surface 221 is greater than the area of the second surface 222, and the area of the first surface 221 is less than or equal to the area of the first cavity wall 151. In the illustrated embodiment, the area of the first surface 221 is less than the area of the first cavity wall 151; the area of the second surface 222 is less than the area of the second cavity wall 152. When the first cavity wall 151 abuts against the first surface 221, the area of the contact surface between the first cavity wall 151 and the first surface 221 is equal to the area of the first surface 221. When the second cavity wall 152 is in contact with the second surface 222, the area of the contact surface between the second cavity wall 152 and the second surface 222 is equal to the area of the second surface 222. The rotor 400 is fixed to the straight shaft part 210. An attraction force is generated between the stator 300 and the rotor 400, which enables the first surface 221 to abut against the first cavity wall 151. In other words, the attraction force between the stator 300 and the rotor 400 causes the raised part 220 to have a tendency to abut against the first cavity wall 151, which can enable the first surface 221 to abut against the first cavity wall 151. Specifically, the direction of the attraction force to the rotor 400 is directed from the second cavity wall 152 to the first cavity wall 151 along the axis of the straight shaft part 210, which can enable the first surface 221 to abut against the first cavity wall 151.

Given that the area of the first surface 221 is greater than that of the second surface 222, the first surface 221 with the larger area can increase the contact area between the first surface 221 and the first cavity wall 151, thereby reducing the pressure per member area of the first surface 221 and the first cavity wall 151, that is, reducing the pressure per member area, thereby reducing the wear of the first surface 221 and the raised part 220.

The raised part 220 further has a side circumferential surface 223. The side circumferential surface 223 connects the first surface 221 and the second surface 222. The side circumferential surface 223 is disposed around the axis of the straight shaft part 210. The annular structure enclosed by the side circumferential surface 223 is coaxial with the straight shaft part 210. The side cavity wall 153 and the side circumferential surface 223 are spaced apart, so that a third gap 163 is defined between the side cavity wall 153 and the side circumferential surface 223. The third gap 163 is in communication with both the first guide groove 114 and the second guide groove 122, so that even when the first surface 221 of the raised part 220 abuts against the first cavity wall 151 of the receiving cavity 150, the third gap 163 can still be in communication with the first through hole 113 via the first guide groove 114. When the second surface 222 of the raised part 220 abuts against the second cavity wall 152 of the receiving cavity 150, the third gap 163 can also be in communication with the second through hole 121 via the second guide groove 122, thereby keeping the flushing liquid flowing smoothly. Specifically, a portion of the first guide groove 114 extends beyond the orthographic projection of the first surface 221 of the raised part 220 on the first cavity wall 151, so as to be in communication with the third gap 163. A portion of the second guide groove 122 extends beyond the orthographic projection of the second surface 222 of the raised part 220 on the second cavity wall 152 to be in communication with the third gap 163.

Referring to FIG. 2, the flushing liquid passes through the first gap 161, the third gap 163, and the second gap 162 in sequence, and flows out of the liquid outlet 32. The flow direction of the flushing liquid is opposite to the flow direction of the blood in the cannula 30, so that the blood in the cannula 30 can be prevented from entering the driving device 20 via the second through hole 121. The thin dotted arrows in FIG. 2 indicate the flow path of the flushing liquid, and the thick dotted lines indicate the flow path of the blood. The first guide groove 114 can not only provide a communication between the first through-hole 113 and the third gap 163, but also enable the flushing liquid to flow better into between the first surface 221 and the first cavity wall 221, which can enable the suspension of the raised part 220 to a certain extent, thereby reducing the abutting pressure between the first surface 221 and the first cavity wall 151, and reducing the wear of the raised part 220. In addition, the flushing liquid flowing into between the first surface 221 and the first cavity wall 221 can also play the role of a lubricant, which can reduce the friction coefficient between the first surface 221 and the first cavity wall 221, and reduce the wear of the raised part 220 and the cavity wall of the receiving cavity 150.

Specifically, a plurality of the first guide grooves 114 are provided. When the number of the first guide grooves 114 increases, on the one hand, the flushing liquid can be filled between the first surface 221 and the first cavity wall 151 in a shorter time, which can lubricate the first surface 221 and the first cavity wall 151, thereby reducing the friction coefficient between the first surface 221 and the raised part 220 to reduce wear. On the other hand, the flow rate and flow velocity of the flushing liquid flowing through the first surface 221 and the first cavity wall 151 can be reasonably increased, which is beneficial to quickly taking away the heat generated by friction between the first cavity wall 151 and the raised part 220, and reducing the serious wear caused by excessive high temperature; on the other hand, the suspension force exerted on the raised part 220 by the flushing liquid can be increased, thereby reducing the abutting pressure between the first cavity wall 151 and the raised part 220, so as to reduce the wear between the first cavity wall 151 and the raised part 220. Therefore, the wear of the first cavity wall 151 and the raised part 220 can be reduced by reasonably increasing the number of the first guide grooves 114. The same principle can be used to reduce the wear of the second surface 222 and the raised part 220 by reasonably increasing the number of the second guide grooves 122. In the illustrated embodiment, four first guide grooves 114 are provided, and the angle between the extending directions of two adjacent first guide grooves 114 is 90°. It can be understood that in other embodiments, the number of the first guide grooves 114 and the number of the second guide grooves 122 can be adjusted as needed.

Specifically, the side circumferential surface 223 includes a cylindrical surface portion 2231 and a tapered surface portion 2232. The cylindrical surface portion 2231 and the tapered surface portion 2232 are arranged along the axis of the straight shaft part 210. The cylindrical surface portion 2231 is connected to the first surface 221 and is perpendicular to the first surface 221. An end of the tapered surface portion 2232 is connected to the cylindrical surface portion 2231, and another end of the tapered surface portion 2232 is connected to the second surface 222, that is, the tapered surface portion 2232 is connected between the cylindrical surface portion 2231 and the second surface 222. Along the axis of the straight shaft part 210 and in the direction from the first surface 221 to the second surface 222, the distance from the cylindrical surface portion 2231 to the axis of the straight shaft part 210 remains constant, and the distance from the tapered surface portion 2232 to the axis of the straight shaft part 210 gradually decreases. The tapered surface portion 2232 provided on the side circumferential surface 223 of the raised part 220 can better guide the flushing liquid. In addition, since the area of the second surface 222 is less than the area of the second cavity wall 152, the flushing liquid flows through the third gap 163 and in turn flows toward the second through hole 121, thereby improving the flushing effect of the flushing liquid. The cylindrical surface portion 2231 has a certain length in the axial direction of the straight shaft part 210, so as to avoid an end of the entire side circumferential surface 223, which is also an end of the tapered raised part 220 adjacent to the first surface 221, from being sharply angled, and in common parlance, avoid the sharp edge from being formed between the side circumferential surface 223 and the first surface 221. When the raised part 220 shakes in the radial direction, if a line-to-surface contact is formed when the edge contacts the side cavity wall 153, it will cause a greater risk of scratching and damage to the side cavity wall 153, and there is no wide enough area for the flushing liquid to form a lubricating film layer between the raised part 220 and the side cavity wall 153, but the provision of the above-mentioned cylindrical surface portion 2231 can play a transitional role, which ensures that the side circumferential surface 223 and the side cavity wall 153 of the receiving cavity 150 are in a surface-to-surface contact, thereby reducing the risk of friction damage.

The shape of the side cavity wall 153 of the receiving cavity 150 is adapted to the shape of the side peripheral surface 223, and has a structure having a vertical surface portion 1531 and an inclined surface portion 1532 that are similar to the cylindrical surface portion 2231 and the tapered surface portion 2232 of the side peripheral surface 223.

Specifically, in the axial direction of the straight shaft part 210, the spacing between the first cavity wall 151 and the second cavity wall 152 is recorded as a first spacing H, the spacing between the first surface 221 and the second surface 222 is recorded as a second spacing h, and the first spacing H is greater than the second spacing h. In some embodiments, the first spacing H is slightly greater than the second spacing h, so that the first cavity wall 151 is always in contact with the first surface 221, and the second cavity wall 152 is always in contact with the second surface 222, thereby preventing the rotating shaft 200 from moving in the axial direction of the straight shaft part 210. In some embodiments, the first spacing H is greater than the second spacing h, so that when the first surface 221 abuts against the first cavity wall 151, the second surface 222 is spaced from the second cavity wall 152 by a distance, so that a gap is formed between the second surface 222 and the second cavity wall 152, which enables a certain floating space of the raised part 220 between the first cavity wall 151 and the second cavity wall 152. As such, the flushing liquid can enter between the first surface 221 and the first cavity wall 151, between the second surface 222 and the second cavity wall 152, and play a role in lubricating and suspending the raised part 220, thereby avoiding dry friction between the raised part 220 and the cavity wall of the receiving cavity 150. Certainly, the difference between the first spacing H and the second spacing h may not be too large, so as to avoid excessive vibration amplitude of the rotating shaft 200 in the axial direction.

Specifically, the width of the gap between the side cavity wall 153 and the tapered surface portion 2232 of the side circumferential surface 223 is greater than the difference between the first spacing H and the second spacing h. That is, the width of the third gap 163 at the position corresponding to the tapered surface portion 2232 is greater than the difference between the first spacing H and the second spacing h. This can prevent the raised part 220 from generating radial and/or axial shaking, thereby reducing the contact probability between the side cavity wall 153 and the side circumferential surface 223 of the raised part 220 and reducing the friction between the raised part 220 and the cavity wall of the receiving cavity 150.

In some embodiments, at least one of the first cavity wall 151 and the first surface 221 is made of ceramic; at least one of the second cavity wall 152 and the second surface 222 is made of ceramic. Ceramics have high processing precision and high biocompatibility, and also have high mechanical strength, good wear resistance and corrosion resistance. The ceramics can have a smaller roughness, which can reduce the friction when the first surface 221 is in contact with the first cavity wall 151, and reduce the friction when the second surface 222 is in contact with the second cavity wall 152. Specifically, the first shaft sleeve 110 and the second shaft sleeve 120 are made of ceramic, and the raised part 220 is made of ceramic, that is, the first cavity wall 151, the first surface 221, the second cavity wall 152, and the second surface 222 are all made of ceramic.

In some embodiments, the outer peripheral surface of the end of the housing assembly 100 adjacent to the impeller 40 forms a liquid guide surface portion 160. The liquid guide surface portion 160 is located in the cannula 30 and corresponds to the position of the liquid outlet 32. The proximal end of the liquid guide surface portion 160 corresponds to the position of the proximal hole wall of the liquid outlet 32. The distance from the liquid guide surface portion 160 to the axis of the straight shaft part 210 gradually increases in the direction away from the impeller 40. Specifically, the liquid guide surface portion 160 is located at the end of the shaft tube 130 away from the pump housing 140. The design of the liquid guide surface portion 160 is beneficial to the discharge of the liquid in the cannula 30. In addition, the impeller 40 and the driving device 20 are usually the rigid parts of the blood pump 1. The shorter the axial length of the rigid part, the more conducive it is to the delivery of the blood pump 1 in the human body. The liquid guide surface portion 160 is disposed on the housing assembly 100 of the driving device 20, so that it is possible to shorten the axial length of the impeller 40 while ensuring the hydraulic performance at the liquid outlet 32. In addition, since the liquid guide surface portion 160 is disposed in the cannula 30 as a part of the housing assembly 100 of the driving device 20, the overall length of the impeller 40 and the driving device 20, (that is, the rigid parts of the blood pump 1) can be reduced, which can further facilitate the delivery of the blood pump 1.

Specifically, the liquid guide surface portion 160 is substantially arc-shaped. Along the axis of the straight shaft part 210, the height L1 of the liquid guide surface portion 160 is 20%-40% of the height L2 of the liquid outlet 32. This height design can shorten the total length of the impeller 40 and the driving device 20 while enabling the blood pump 1 to have better hydraulic performance.

Referring to FIGS. 2 and 6, the stator 300 is fixedly received in the pump housing 140. Specifically, the stator 300 includes a magnetic core 310, a back plate 320, and a coil 330. The back plate 320 is fixedly connected to the pump housing 140. A plurality of magnetic cores 310 are provided. The plurality of magnetic cores 310 are spaced apart along a circumference. The extending direction of each magnetic core 310 is in the same direction as the extending direction of the straight shaft part 210, that is, the central axis of the magnetic core 310 is parallel to the axis of the straight shaft part 210. An end of each magnetic core 310 is fixedly connected to the back plate 320. The number of coils 330 is the same as the number of magnetic cores 310, and the coils 330 and the magnetic cores 310 are in a one-to-one correspondence. The coils 330 are wound around the magnetic cores 310, so that each magnetic core 310 is wound with one coil 330.

In some embodiments, the magnetic core 310 includes a magnetic post 311 and a head (i.e., a pole shoe) disposed at an end of the magnetic post 311. A size of a cross section of the head is greater than a size of a cross section of the magnetic post 311. The extending direction of the magnetic post 311 is in the same direction as with the extending direction of the straight shaft part 210. The back plate 320 is engaged with the end of the magnetic post 311 away from the head. In the extending direction of the magnetic post 311, the magnetic post 311 is roughly a cylindrical body with uniform size, that is, the size of the cross section of the magnetic post 311 remains constant, and in common parlance, the thickness of the magnetic post 311 is uniform. The coil 330 is wound around the magnetic post 311 of the magnetic core 310. In the illustrated embodiment, the magnetic core 310 only includes the magnetic post 311, that is, the magnetic core 310 does not have a head (i.e., a pole shoe) with a larger width, then the magnetic post 311 of the stator 300 is the magnetic core 310. In this case, the entire magnetic core 310 can be magnetically coupled with the rotor 400. Compared with the magnetic core 310 with the pole shoe, the magnetic core 310 having only the magnetic post 311 can, on the one hand, reduce magnetic loss and increase the magnetic coupling density between the magnetic core 310 and the rotor 400, so as to increase the torque of the stator 300 on the rotor 400 under the same current. On the other hand, the magnetic cores 310 without heads can also greatly reduce the problem of power reduction of the driving device 20 caused by local magnetic short circuits due to contact between adjacent magnetic cores 310.

It can be understood that the magnetic core 310 is not limited to have the above two configurations. In some embodiments, some of the magnetic posts 311 are provided with heads, while other parts of the magnetic posts 311 are not provided with heads.

In some embodiments, the cross-section of the magnetic post 311 is roughly in the shape of a triangular prism, and one edge of each magnetic post 311 faces the axis of the straight shaft part 210. In some embodiments, the edges of the magnetic post 311 are all chamfered, that is, the edges of the magnetic post 311 are relatively smooth and blunt chamfered edges, so as to eliminate the sharp edges and corners on the magnetic post 311, which not only facilitates the subsequent winding of the coil 330, but also helps to protect the insulating material coated on the coil 330. In other embodiments, the cross section of the magnetic post 311 can also be in the shape of a sector, a circle, a trapezoid, a sector ring, etc.

The back plate 320 is roughly a flat plate structure. The back plate 320 is made of the same material as the magnetic core 310, such as cobalt steel and other soft magnetic materials. Taking the rotor 400 driven by the stator 300 as a reference, the back plate 320 is fixed to the end of the magnetic post 311 away from the rotor 400. The back plate 320 can play the role of closing the magnetic circuit of the stator 300 to promote and increase the generation of magnetic flux of the stator 300 and improve the coupling ability between the stator 300 and the rotor 400. In other words, the back plate 320 is disposed in the stator 300 to promote and increase the generation of magnetic flux of the stator 300 and improve the coupling ability between the stator 300 and the rotor 400. Since the back plate 320 can increase the magnetic flux, the back plates 320 are respectively disposed in the stator 300, which is also conducive to reducing the overall diameter of the driving device 20. It can be understood that in some embodiments, the back plate 320 can also be omitted.

The rotor 400 and the stator 300 are spaced apart along the axis of the straight shaft part 210. Along the axis of the straight shaft part 210, the rotor 400 is located between the raised part 220 and the stator 300. The first cavity wall 151 of the receiving cavity 150 is located between the rotor 400 and the first surface 221 of the raised part 220.

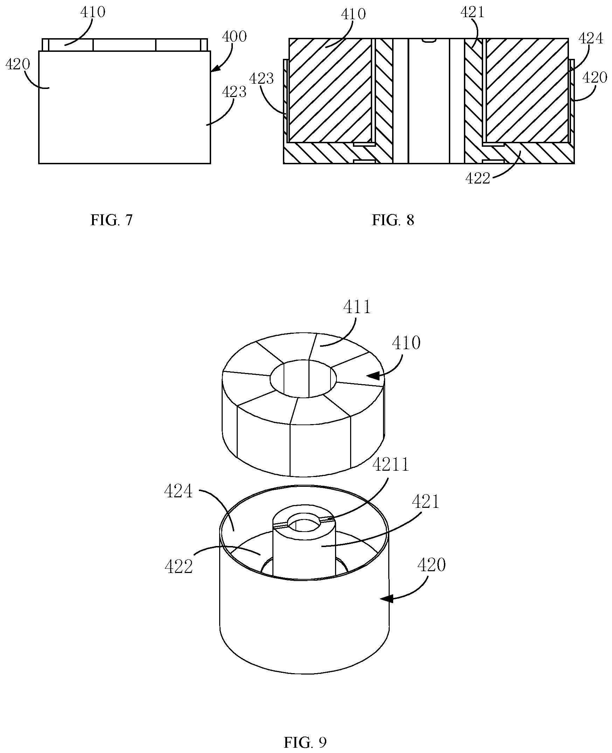

Referring to FIGS. 7, 8 and 9, the rotor 400 is magnetic, and the stator 300 can generate a rotating magnetic field to drive the rotor 400 to rotate. An attractive force is generated between the rotor 400 and the magnetic core 310. Specifically, the rotor 400 includes a magnet 410. The magnet 410 is fixed to the straight shaft part 210 of the rotating shaft 200. The magnetic core 310 of the stator 300 has an attractive force on the magnet 410 of the rotor 400. Such attractive force directs from the second surface 222 to the first surface 221 along the axis of the rotating shaft 200, which can enable the first surface 221 to abut against the first cavity wall 151.

The magnet 410 is a ring-shaped Halbach array magnet. Specifically, the magnet 410 includes a plurality of magnetic members 411 magnetized along the axial direction of the magnet 410, for example, four, six, eight or ten, etc., magnetic members 411 may be provided. Each magnetic member 411 is in a sector ring shape, and the plurality of magnetic members 411 are disposed around the straight shaft part 210 in a circle to form the magnet 410 into an annular structure.

The rotor 400 further includes a flywheel 420. In this case, the flywheel 420 is directly fixed on the straight shaft part 210, and the magnet 410 is fixed on the flywheel 420. The flywheel 420 can enhance the connection strength between the magnet 410 and the straight shaft part 210. In addition, the shaking of the rotating shaft 200 during the rotation process can be reduced, making the entire rotating shaft 200 more stable during the rotation process.

The flywheel 420 includes a built-in tube 421, a disc-shaped part 422, and an outer annular wall 423. Both the built-in tube 421 and the outer annular wall 423 have circular tubular structures. The disc-shaped part 422 is an annular disc structure. The built-in tube 421 and the outer annular wall 423 are both fixedly connected to the disc-shaped part 422. The outer annular wall 423 is disposed around the disc-shaped part 422. The built-in tube 421 and the outer annular wall 423 are coaxial. The straight shaft part 210 is inserted in the built-in tube 421 and fixedly connected to the built-in tube 421. A mounting cavity 424 is formed between the built-in tube 421 and the outer annular wall 423. The mounting cavity 424 is an annular cavity. The magnet 410 is respectively received in the mounting cavity 424. The shape of the mounting cavity 424 is adapted to the magnet 410 to facilitate the mounting and positioning of the magnet 410. Such a configuration enables the flywheel 420 to limit the magnet 410, which not only facilitates the mounting of the magnet 410, but also makes the connection between the magnet 410 and the flywheel 420 more stable.

It should be noted that the flywheel 420 is not limited to have the above structure. In some embodiments, the outer annular wall 423 of the flywheel 420 is omitted. In some embodiments, the outer annular wall 423 and the built-in tube 421 of the flywheel 420 are omitted. In this case, the straight shaft part 210 fixedly extends through a center of the disc-shaped part 422. Compared with the flywheel 420 having only the disc-shaped part 422, the disposition of the built-in tube 421 can enable the flywheel 420 to be connected to the straight shaft part 210 more stably.

In order to facilitate the mounting of the magnet 410 and improve the mounting accuracy of the magnet 410, the flywheel 420 is further provided with an identification part 4211 configured for determining the mounting position of the magnetic member 411. The identification part 4211 can be configured as a groove, a scale line or an identification, etc. When mounting the magnet 410, as long as the identification part 4211 is used to respectively identify the position of one of the magnetic members 411 of the magnet 410, the mounting positions of the remaining magnetic members 411 can be determined, thereby facilitating the mounting of the magnet 410. Specifically, the identification part 4211 is disposed on at least one of the built-in tube 421, the disc-shaped part 422 and the outer annular wall 423. For example, the identification part 4211 is disposed on the end surface of the built-in tube 421.

In the illustrated embodiment, the rotating shaft 200 and the stator 300 are spaced apart along the axis of the straight shaft part 210, that is, the straight shaft part 210 is not inserted into the stator 300, so that the rotating shaft 200 is located outside the stator 300. Since an area of the cross section of the magnetic post 311 is increased, and the greater the area of the cross section of the magnetic post 311, the greater the magnetic flux generated, the greater the torque of the stator 300 on the rotor 400, and the smaller the required current, this helps reduce power consumption and heat generation. Since the rotating shaft 200 is not inserted into the stator 300, the rotating shaft 200 can be prevented from occupying the mounting space of the magnetic post 311, which is beneficial for increasing the size of the cross section of the magnetic post 311 of the stator 300 while keeping the outer diameter of the housing assembly 100 unchanged to increase the driving torque of the stator 300 to the rotor 400. With the same required torque, this method can reduce the current supply to the stator 300, thereby reducing power consumption and reducing the heat generated by the driving device 20, so as to avoid discomfort or even harm to the human body due to excessively high temperature caused by the heat accumulation during the operation of the blood pump 10.

The above-mentioned driving device 20 and blood pump 1 have at least the following advantages.
(1) Since the attractive force is generated between the stator 300 and the rotor 400 of the driving device 20, the attractive force causes the first surface 221 to abut against the first cavity wall 151 of the receiving cavity 150, so that the first cavity wall 151 is subjected to the pressure of the first surface 221. By setting the area of the first surface 221 to be greater than the area of the second surface 222, that is, increasing the area of the first surface 221, and setting the area of the first surface 221 to be less than or equal to the area of the first cavity wall 151, when the first surface 221 contacts the first cavity wall 151, the contact area between the first surface 221 and the first cavity wall 151 is equal to the area of the first surface 221. The first surface 221 with a larger area can increase the contact area between the first surface 221 and the first cavity wall 151 when the first surface 221 contacts the first cavity wall 151, thereby reducing the pressure per member area between the first surface 221 and the first cavity wall 151. That is, the intensity of pressure per member area is reduced, thereby reducing the wear of the first surface 221 and the first cavity wall 151. In addition, since the attraction force can cause the first surface 221 to abut against the first cavity wall 151 of the receiving cavity 150, the second surface 222 tends to move away from the second cavity wall 152, so that the second surface 222 and the second cavity wall 152 are not in contact with each other, or the friction coefficient when the second surface 222 and the second cavity wall 152 are in contact with each other is reduced. During the startup process of the driving device 20, the friction resistance of the second cavity wall 152 to the raised part 220 can be reduced, thereby increasing the startup speed of the rotation of the rotating shaft 200, that is, increasing the sensitivity of the rotating shaft 200 responsive to the driving. Therefore, the above-mentioned blood pump 1 and the driving device 20 can not only reduce the degree of wear of the rotating shaft 200 during use, but also can be started faster.
(2) By disposing the first guide groove 122 on the first surface 221, the flushing liquid can quickly flow into the space between the first surface 221 and the first cavity wall 151, so as to play a lubricating role between the first surface 221 and the first cavity wall 151, and reduce the friction coefficient between the first surface 221 and the first cavity wall 221, so as to reduce the wear of the raised part 220 and the cavity wall of the receiving cavity 150. Further, a portion of the first guide groove 114 is located outside the orthographic projection of the first surface 221 of the raised part 220 on the first cavity wall 151, so that even when the first surface 221 of the raised part 220 abuts against the first cavity wall 151 of the receiving cavity 150, the receiving cavity 150 can be in communication with the first through hole 113 via the first guide groove 114, so as to ensure the smooth flow of the flushing liquid.
(3) A portion of the housing assembly 100 is disposed in the cannula 30, and the arc-shaped liquid guide surface portion 160 is disposed on the outer peripheral surface of the portion of the housing assembly 100 located in the cannula 30. This is beneficial for reducing the overall length of the impeller 40 and the driving device 20 (i.e., the rigid parts of the blood pump 1) while ensuring the hydraulic performance of the blood pump 1, so as to facilitate the delivery of the blood pump 1.
(4) By spacing the rotating shaft 200 and the stator 300, it is beneficial to increase the driving torque of the stator 300 on the rotor 400 by increasing the area of the cross section of the magnetic post 311 while keeping the outer diameters of the housing assembly 100 and the stator 300 unchanged. With the same required torque, this method can reduce the current supply to the stator 300, thereby reducing power consumption and reducing the heat generated by the driving device 20, so as to avoid discomfort or even harm to the human body due to excessively high temperature caused by the heat accumulation during the operation of the blood pump 10.

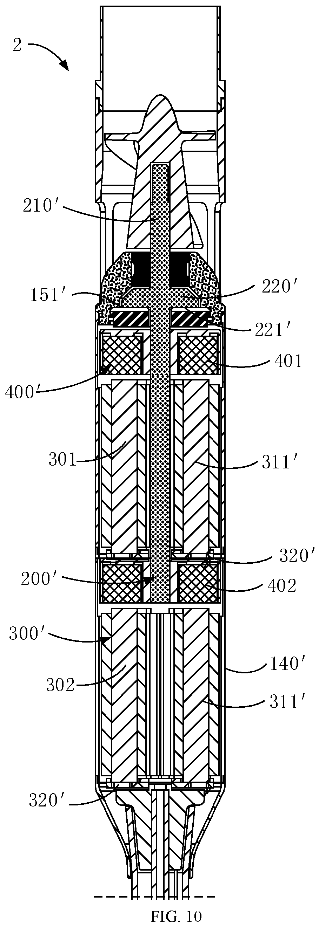

Referring to FIG. 10, the blood pump 2 of the second embodiment has substantially the same configuration as the blood pump 1 of the first embodiment, except that, in this embodiment, the rotor 400' has two rotor members, the stator 300' has two stator members. The two stator members are respectively named as a first stator member 301 and a second stator member 302. The two rotor members are respectively named as a first rotor member 401 and a second rotor member 402.

The first rotor member 401, the first stator member 301, the second rotor member 402, and the second stator member 302 are arranged in sequence along the axis of the straight shaft part 210'. The first rotor member 401 is disposed closest to the raised part 220'. The first rotor member 401 and the second rotor member 402 are both fixedly connected to the straight shaft part 200' of the rotating shaft 200'. An attraction force is generated between the first stator member 301 and the first rotor member 401, and an attraction force is generated between the second stator member 302 and the second rotor member 402. The attraction force of the first stator member 301 to the first rotor member 401 is recorded as a first attraction force, and the attraction force of the second stator member 302 to the second rotor member 402 is recorded as a second attraction force. The directions of the first attraction force and the second attraction force are the same. The first attraction force and the second attraction force act on the rotating shaft 200' through the first rotor member 401 and the second rotor member 402 respectively. Therefore, under the combined force formed by the first attraction force and the second attraction force, the first surface 221' of the raised part 220' can be cause to abut against the first cavity wall 151'.

The straight shaft part 210' of the rotating shaft 200' can rotatably extend through the first stator member 301 and is spaced apart from the second stator member 301. That is, the straight shaft part 210' is not inserted into the second stator member 302, so that the straight shaft part 210' is located outside the second stator member 302, and then the rotating shaft 200' and the second stator member 302 are spaced apart by a certain distance in the axial direction of the straight shaft part 210'. The first stator member 301 and the second stator member 302 each have a magnetic post 311'. The size of the cross section of the magnetic post 311' of the second stator member 302 is greater than the size of the cross section of the magnetic post 311' of the first stator member 301. Since the rotating shaft 200' is not inserted into the second stator member 302, that is, the rotating shaft 200' is located outside the second stator member 302, it is possible to avoid the rotating shaft 200' occupying the mounting space of the magnetic post 311' in the second stator member 302, and to increase the size of the cross section of the magnetic post 311' of the second stator member 302 without increasing the outer diameters of the pump housing 140' and the second stator member 302. In this case, although the outer diameters of the first stator member 301 and the second stator member 302 are the same, the size of the cross section of the magnetic post 311' of the second stator member 302 is greater than size of the cross section of the magnetic post 311' of the first stator member 301. In this way, the driving torque of the second stator member 302 on the second rotor member 402 can be increased. With the same required torque, this method can reasonably reduce the current supply to the second stator member 302, thereby reducing power consumption and reducing the heat generated by the driving device, so as to avoid discomfort or even harm to the human body due to excessively high temperature caused by the heat accumulation during the operation of the blood pump.

In this embodiment, the first rotor member 401 and the second rotor member 402 may have configurations similar to the that of the rotor 400 of the blood pump 1 of the first embodiment; the first stator member 301 and the second stator member 302 may have configurations similar to the that of the stator 300 of the blood pump 1, and which will not be repeatedly described in detail herein. The back plate 320' of the first stator member 301 is located at an end of the magnetic post 311' of the first stator member 301 away from the first rotor member 401, and the back plate 320' of the second stator member 302 is located at an end of the magnetic post 311' of the second stator member 302 away from the second rotor member 402.

The blood pump 2 of the second embodiment have a configuration similar to that of the blood pump 1 of the first embodiment. Therefore, the blood pump 2 of the second embodiment also has the advantages of the blood pump 1 of the first embodiment.

It can be understood that the configuration of the driving device of the blood pump is not limited to the configurations of the first embodiment and the second embodiment. In other embodiments, the number of stator members can be adjusted as needed, and the positional relationship between the rotor member and the stator member can also be adjusted.

The above embodiments are only used to illustrate the technical solutions of the present application, rather than to limit the same. Although the present application has been described in detail with reference to the aforementioned embodiments, those skilled in the art should understand that the technical solutions described in the aforementioned embodiments may still be modified, or some of the technical features may be replaced by equivalents. Such modifications or replacements do not deviate the essence of the corresponding technical solutions from the spirit and scope of the technical solutions of the embodiments of the present application, and shall all be included in the protection scope of the present application.

## Claims

1. A driving device, configured to drive an impeller to rotate, comprising:
a housing assembly provided with a receiving cavity, wherein the receiving cavity has a first cavity wall and a second cavity wall that face each other and are spaced apart from each other;
a rotating shaft configured to be connected to the impeller, wherein the rotating shaft comprises a straight shaft part and a raised part that are connected to each other, the raised part protrudes from the straight shaft part in a circumferential direction of the straight shaft part; the raised part is rotatably received in the receiving cavity; the raised part is located between the first cavity wall and the second cavity wall and has a first surface and a second surface; the first surface faces the first cavity wall, the second surface faces the second cavity wall; an area of the first surface is greater than an area of the second surface, and the area of the first surface is less than or equal to an area of the first cavity wall;
a rotor fixedly connected to the straight shaft part; and
a stator configured to drive the rotor to rotate,
wherein an attractive force is generated between the stator and the rotor, and the attractive force enables the first surface to abut against the first cavity wall.

2. The driving device according to claim 1, wherein a spacing between the first cavity wall and the second cavity wall is greater than a spacing between the first surface and the second surface, so that when the first surface abuts against the first cavity wall, the second surface and the second cavity wall are spaced apart by a distance.

3. The driving device according to claim 1, wherein at least one of the first cavity wall and the first surface is made of ceramic;
and/or, at least one of the second cavity wall and the second surface is made of ceramic.

4. The driving device according to claim 1, wherein the first surface and the second surface are both perpendicular to an axis of the straight shaft part; and the first cavity wall and the second cavity wall are parallel to the first surface and the second surface respectively.

5. The driving device according to claim 4, wherein outer contours of the first surface and the second surface are circular, and a center line of the first surface and a center line of the second surface are coaxial with the axis of the straight shaft part.

6. The driving device according to claim 1, wherein a first through hole and a first guide groove are formed on the first cavity wall; the first through hole is in communication with the receiving cavity; the first guide groove is in communication with the first through hole and the receiving cavity; and the straight shaft part rotatably extends through the first through hole.

7. The driving device according to claim 6, wherein the raised part further has a side circumferential surface connecting the first surface and the second surface; the receiving cavity further has a side cavity wall connecting the first cavity wall and the second cavity wall; a gap is formed between the side cavity wall and the side circumferential surface; and a portion of the first guide groove extends beyond an orthographic projection of the first surface on the first cavity wall and is in communication with the gap.

8. The driving device according to claim 7, wherein the side circumferential surface comprises a cylindrical surface portion and a tapered surface portion that are disposed around an axis of the straight shaft part, wherein the cylindrical surface portion is connected to the first surface, and the tapered surface portion is connected between the cylindrical surface portion and the second surface; a distance from the tapered surface portion to the axis of the straight shaft part gradually decreases in a direction from the first surface to the second surface; a spacing between the first cavity wall and the second cavity wall is defined as a first spacing, a spacing between the first surface and the second surface is defined as a second spacing, and a width of a gap between the side cavity wall and the tapered surface portion is greater than a difference between the first spacing and the second spacing.

9. The driving device according to claim 8, wherein the side cavity wall comprises a vertical surface portion and an inclined surface portion that are connected to each other; a shape of the vertical surface portion is adapted to a shape of the cylindrical surface portion; and a shape of the inclined surface portion is adapted to a shape of the tapered surface portion.

10. The driving device according to claim 6, wherein an end of a hole wall of the first through hole adjacent to the receiving cavity is provided with a chamfer.

11. The driving device according to claim 1, wherein a second through hole and a second guide groove are disposed on the second cavity wall; the second through hole is in communication with the receiving cavity; the second guide groove is in communication with the second through hole and the receiving cavity; the straight shaft part rotatably extends through the second through hole; and a portion of the second guide groove extends beyond an orthographic projection of the second surface on the second cavity wall.

12. The driving device according to claim 1, wherein the housing assembly comprises a shaft tube, a first shaft sleeve, and a second shaft sleeve that jointly enclose the receiving cavity; wherein the first shaft sleeve and the second shaft sleeve are spaced apart and fixed in the shaft tube; the first cavity wall is located on the first shaft sleeve; the second cavity wall is located on the second shaft sleeve; and the straight shaft part rotatably extends through the first shaft sleeve and the second shaft sleeve.

13. The driving device according to claim 1, wherein the rotor and the stator are spaced apart along an axis of the straight shaft part; along the axis of the straight shaft part, the rotating shaft and the stator are spaced apart; the stator comprises a magnetic core and a coil wound around the magnetic core; the rotor is magnetic; and an attractive force is generated between the rotor and the magnetic core.

14. The driving device of claim 1, wherein the first cavity wall is located between the rotor and the first surface.

15. The driving device according to claim 1, wherein the rotating shaft and the stator are spaced apart from each other along an axis of the straight shaft part.

16. The driving device according to claim 1, wherein the rotor comprises a first rotor member and a second rotor member; the stator comprises a first stator member and a second stator member, wherein the first rotor member, the first stator member, the second rotor member and the second stator member are arranged in sequence along an axis of the straight shaft part; the first rotor member is closest to the raised part; the first stator member is configured to generate a rotating magnetic field to drive the first rotor member to rotate; the second stator member is configured to generate a rotating magnetic field to drive the second rotor member to rotate; the straight shaft part rotatably extends through the first stator member and is spaced apart from the second stator member; the first stator member and the second stator member each have a magnetic post; and a size of a cross section of the magnetic post of the second stator member is greater than a size of a cross-section of the magnetic post of the first stator member.

17. A blood pump, comprising an impeller and a driving device configured to drive an impeller to rotate, the driving device comprising:
a housing assembly provided with a receiving cavity, wherein the receiving cavity has a first cavity wall and a second cavity wall that face each other and are spaced apart from each other;
a rotating shaft comprising a straight shaft part and a raised part that are connected to each other, wherein the straight shaft part is connected to the impeller; the raised part protrudes from the straight shaft part in a circumferential direction of the straight shaft part; the raised part is rotatably received in the receiving cavity; the raised part is located between the first cavity wall and the second cavity wall and has a first surface and a second surface; the first surface faces the first cavity wall, the second surface faces the second cavity wall; an area of the first surface is greater than an area of the second surface, and the area of the first surface is less than or equal to an area of the first cavity wall;
a rotor fixedly connected to the straight shaft part; and
a stator configured to drive the rotor to rotate,
wherein an attractive force is generated between the stator and the rotor, and the attractive force enables the first surface to abut against the first cavity wall.

18. The blood pump according to claim 17, further comprising a cannula connected to the housing assembly, wherein a liquid outlet is defined on a wall of the cannula; the impeller is rotatably disposed in the cannula; the impeller is disposed adjacent to the liquid outlet; the straight shaft part is partially received in the housing assembly, and partially received in the cannula and fixedly connected to the impeller; an outer peripheral surface of an end of the housing assembly adjacent to the impeller forms a liquid guide surface portion; the liquid guide surface portion is located in the cannula and corresponds to a position of the liquid outlet; a proximal end of the liquid guide surface portion corresponds to a position of a proximal hole wall of the liquid outlet; in a direction away from the impeller, a distance from the liquid guide surface portion to an axis of the straight shaft part gradually increases.

19. The blood pump according to claim 18, wherein along the axis of the straight shaft part, a height of the liquid guide surface portion is 20%-40% of a height of the liquid outlet.

20. The driving device according to claim 17, wherein the housing assembly comprises a shaft tube, a first shaft sleeve, and a second shaft sleeve that jointly enclose the receiving cavity, wherein the first shaft sleeve and the second shaft sleeve are spaced apart and fixed in the shaft tube; the first cavity wall is located on the first shaft sleeve; the second cavity wall is located on the second shaft sleeve; and the straight shaft part rotatably extends through the first shaft sleeve and the second shaft sleeve;
an outer peripheral surface of an end of the shaft tube adjacent to the impeller forms the liquid guide surface portion.
